# EUROPEAN PATENT APPLICATION

(11) **EP 2 548 457 A1**
(43) Date of publication of application: **23.01.2013**
(21) Application number: 11005880.7
(22) Date of filing: 18.07.2011
(51) Int. Cl.: A23L 1/305, A23C 1/04, A23C 1/08, A23C 7/04, A23C 9/18, A23C 9/20, A61P 37/08

(54) **Raw milk preparation for use for preventing or treating asthma and other allergic diseases in infants and children**

(71) Applicant: Ludwig-Maximilians-Universität München, 80539 München (DE)
(72) Inventor: Prof. Dr. von Mutius, Erika, 82319 Leutstetten (DE); Prof. Dr. Braun-Fahrländer, Charlotte, 4002 Basel (CH); Prof. Dr. Kneifel, Wolfgang, 1190 Wien (AT)
(74) Representative: Leissler-Gerstl, Gabriele

(57) **Abstract**

Disclosed in the application is a raw milk preparation for use for treating or preventing childhood asthma and allergic diseases and methods of producing the raw milk preparation for use for treating or preventing childhood asthma and allergic diseases. Also disclosed is the use of the a raw milk preparation as nutritional supplement for a female human being during pregnancy, as a preparation for a planned pregnancy and/or during nursing of the infant or for an infant or child from birth to at least until completion of the 1^{st} or 3^{rd} year of age.

## Description

### Field of invention

The present invention relates to the treatment and prevention of asthma and allergic diseases.

### Background

Childhood asthma, and allergic diseases remain a major health problem in industrialized countries and increasingly in developing countries. Asthma's prevalence is increasing, especially among children, highlighting the need for effective treatments and prevention. Study populations with a similar genetic background but striking differences in environmental exposures have been especially informative to clarify environmental causes for the onset of asthma and atopy. Studies focusing on differences between rural farming and non-farming communities have consistently shown that children growing up on a farm are at significantly lower risk to develop asthma, hay fever, and atopic sensitisation than children living in the same rural area but not on a farm.

Exposure to farming or the farming lifestyle in early life (Riedler et al. Exposure to farming in early life and development of asthma and allergy: a cross-sectional survey. Lancet 2001;358(9288):1129-33; Perkin MR, Strachan DP. Which aspects of the farming lifestyle explain the inverse association with childhood allergy? J Allergy Clin Immunol 2006;117(6):1374-81) have been associated with a reduced asthma and atopy risk.

Environmental factors that have been hypothesised to explain this protective effect of farm life are contact with animals, endotoxin levels in house dust, and farm milk consumption.

People living on a farm, who are consuming raw milk, are therefore very likely subjected to increased exposure to bacteria and fungi due to the presence of farm animals such as pigs, horses and cows, and the microbial organisms present in raw farm milk, because raw farm milk has not been processed by purification and pasteurisation processes that involve thermal or mechanical treatment and, therefore, has a higher load of microbial organisms.

According to the hygiene hypothesis, this exposure to microorganisms during early childhood or even during embryogenesis may explain the low prevalence of asthma and other allergic diseases associated with "farming lifestyle".

The hygiene hypothesis suggests that a newborn baby's immune system must be educated to function properly during infancy and the rest of life. Allergic and autoimmune diseases can be the result of an immune system not functioning properly.

The "hygiene hypothesis" is supported by epidemiologic studies demonstrating that allergic diseases and asthma are more likely to occur when the incidence and levels of endotoxin (bacterial lipopolysaccharide, or LPS) in the home are low. LPS is a bacterial molecule that stimulates and educates the immune system.

However, this hypothesis has not been proven and it remains unclear, what aspect of the farming lifestyle causes the low prevalence of asthma and other allergic diseases.

On the other hand, allergies to dairy products are very common, especially amongst children under the age of 3. Milk allergy is the most common food allergy in early childhood. It affects somewhere between 2% and 3% of infants in developed countries, but approximately 85-90% of affected children lose clinical reactivity to milk once they surpass 3 years of age. (Host A (December 2002). "Frequency of cow's milk allergy in childhood". Ann. Allergy Asthma Immunol. 89 (6 Suppl 1): 33-7).

The allergens are the milk proteins. Many times the allergen is casein. But whey proteins have also been identified as being associated as allergens. Several whey proteins, like α-lactalbumin and β-lactoglobulin are known as major allergens.

This has spurred a whole new industry providing alternatives for bovine milk, like for example goat's milk, sheep's milk, soy milk, rice milk and almond milk.

These sorts of milk are substitutes for people afflicted with allergies to whey proteins, as the whey proteins are not present in these milks.

It was the object of the present invention to provide a preparation for the prevention and/or treatment of asthma and allergic diseases in infants and children.

### Summary of the invention

The inventors of the present invention surprisingly found that a raw milk preparation comprising undenatured whey proteins effects the decrease of the prevalence of asthma and allergic diseases. It could be shown that regular consumption of a raw milk preparation comprising undenatured whey proteins is inversely associated with asthma, hay fever and atopy, atopic dermatitis and food allergen sensitisation. Early exposure and daily consumption showed a stronger inverse association. More surprisingly, it was found that the microbial load of the raw milk preparation was not related to the clinical health outcome. Thus, the inventors could identify part of the farming lifestyle, which is responsible for the low prevalence of asthma and allergic diseases in infants and children, and they provided a preparation for use for treating and preventing asthma and allergic diseases.

Therefore, it is the gist of the present invention to use a raw milk preparation, particularly a raw milk preparation as defined below, for treating or preventing asthma and allergic diseases. Most preferably, the raw milk preparation is used for treating or preventing asthma. The raw milk preparation is particularly effective in infants and children in an age where the immune system is in development. Preferably, the raw milk preparation according to the invention is used for treatment or prevention of asthma or allergic diseases in infants and children until completion of the 3^{rd} year of age.

As asthma and allergic diseases also occur in other mammalian species besides human beings, it is also contemplated to use the raw milk preparation of the present invention for the prevention or treatment of asthma and allergic diseases in mammals. Such mammals can be, without being limited to the explicitly mentioned examples, equines, felines and canines, such as horses, cats and dogs.

Moreover, it has been found that the raw milk preparation of the present invention as defined and described below and in the claims can be used with advantage already before the infant is born, and, thus, the present invention provides also a raw milk preparation for use for treatment and particularly prevention of asthma or allergic diseases in infants by administration of the untreated raw milk to a female human being, who is pregnant, nursing or preparing for a planned pregnancy.

In another embodiment of the present invention the raw milk preparation as defined and described below is used as a nutritional supplement for a female human being during pregnancy, as a preparation for a planned pregnancy and/or during nursing of the infant or for an infant or child after birth. It has been found that administering the raw milk preparation of the present invention in the first 3, preferably at least 6 months and more preferably at least until completion of the 1st year, more preferably until completion of the 3^{rd} year, has a beneficial effect for the health of the infant or child.

### Definitions

The term "raw milk preparation" when used in this specification can refer to either native raw milk (raw whole milk), a preparation comprising at least the whey and fat fraction (whey-fat-preparation) of native raw milk, or native raw milk or a whey-fat-preparation in dehydrated form, in particular in spray dried or freeze dried form. The "raw milk preparation" of the present invention contains heat-sensitive proteins in native, undenatured form.

In the present application the term "untreated" is used for a raw milk preparation that has not been subjected to any treatment that causes damage to the heat-labile milk ingredients such as the heat-labile proteins in the whey. A "raw milk preparation" being untreated means that the raw milk preparation of the present invention has not been subjected to heat treatment and/or mechanical treatment steps that are usually used in dairy to decontaminate, standardize or sterilize the milk and adapt the fat content. In this regard "mechanical treatment" methods shall refer to methods like homogenisation, which introduce mechanical strain, and thereby destroy the structure of milk components. Methods that do not modify the structure of milk components, in particular the structure of proteins or the fat globules, like filtration methods, are not comprised by the term "mechanical treatment". "Heat treatment method" shall refer to any heating step wherein milk is heated to temperatures above 55°C for at least 20 seconds at atmospheric pressure.

"Milk" as used in the present description can be milk from any mammal, such as cattle, sheep or goat. Preferably the milk is bovine milk.

The term "whey proteins" in the context of the present invention refers to those proteins that are present in the whey, i.e. are found in the aqueous phase of milk after precipitation of the casein fraction. The term "protein" as used in the present description shall comprise proteins and peptides.

"Denaturation" is a process in which proteins lose at least their quaternary structure, or also their tertiary structure and includes processes which lead up to the loss of the secondary structure by application of some external stress or of a compound, such as a strong acid or base, a concentrated inorganic salt, an organic solvent, or by heat. "External stress" as used above, which leads to denaturation, can be exerted by other means than mechanical stress or treatment. "Denatured proteins" in the present description are proteins that have been damaged by external stress, i.e. have lost at least part of their native structure. An undenatured protein in the context of the present invention displays a substantially undamaged structure.

The state of denaturation of a protein can be determined with methods known in the art such as antibody assays, electrophoretic, chromatographic or enzymatic methods.

A significant distinction between treated milk and the raw milk preparation of the present invention is the amount of denatured whey proteins comprised in the milk and the amount of active enzymes present in the milk. Decrease of enzyme activity, particularly of lactoperoxidase and/or alkaline phosphatase activity, can be detected via methods known to the person skilled in the art.

Furthermore, the level of denaturation can be assessed by a person skilled in the art by comparing the level of undenatured protein in the raw milk preparation with the level of undenatured protein in a milk preparation after a denaturing treatment. The distinction between denatured and undenatured protein can be assured by using, for example, an antibody that only binds to the undenatured protein displaying its native secondary and tertiary structure and/or by standardized HPLC (High pressure/performance liquid chromatography) methods. The existence of a native quaternary structure of proteins can be determined using methods for directly or indirectly measuring mass and/or size of the protein complex. Those methods are known to the person skilled in the art and comprise, for example, mass spectrometric immunoassay and size-exclusion chromatography.

The denaturation process can be in part a proteolytic process. The resulting degradation products can be detected via methods known to the person skilled in the art.

Thermal stress or treatment leads to the formation or loss or restructuring of disulfide bridges in the proteins comprised in milk. These processes can be reversed by reduction agents. These agents can be detected as well.

The raw milk preparation of the present invention contains at least about 75%, preferably at least about 80, more preferably at least about 90%, even more preferred at least about 95 and ideally at least 98 % of the whey proteins in undamaged form. The percentage of damage can be determined with well-known methods as disclosed above.

"Asthma" is a common chronic disorder of the airways that is complex and characterized by variable and recurring symptoms, airflow obstruction, bronchial hyper-responsiveness, and an underlying inflammation. Its prevalence is increasing, especially among children. Asthma comprises current and/or chronic asthma. Two major environmental factors have emerged as the most important in the development, persistence, and possibly severity of asthma: airborne allergens and viral respiratory infections. Allergens cause allergies. Therefore it is beneficial for the treatment or prevention of asthma to include the treatment of allergic diseases as well.

The terms "allergic diseases" or "allergy" are used interchangeably throughout the description of this invention. Allergy is a hypersensitivity disorder of the immune system. Allergies are the result of inappropriate immune responses to normally harmless substances. Allergy symptoms vary widely, from sneezing, watery eyes, and nasal congestion of mild "hay fever" to severe rashes, swelling, and shock. Allergic diseases include without being limited to hay fever, atopy and atopic dermatitis.

### Figures

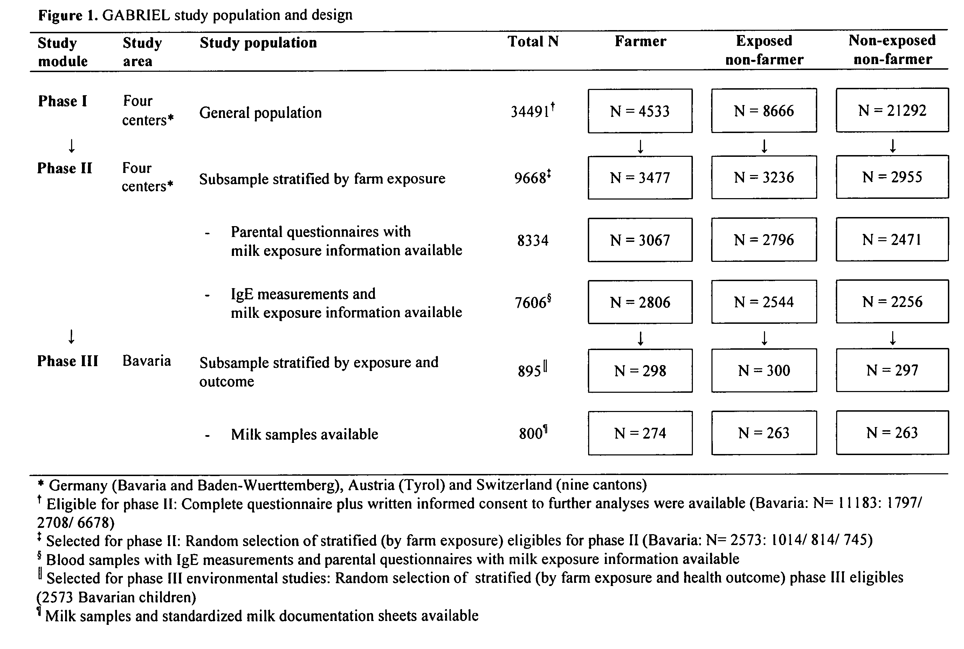

Figure 1 shows the GABRIELA (an advanced multidisciplinary study to identify the genetic and environmental causes of asthma in the European Community) study population and design.
Figure 2 shows the milk exposure of farmers and non-farmers in Phase II and III.
Figure 3 shows the adjusted association of reported milk exposure and asthma, atopy, hay fever and atopic dermatitis (Phase II).
Figure 4 shows levels and percentage of detectables of all milk constituents stratified by milk type and milk's heating status.
Figure 5 shows the adjusted association of asthma or atopy and milk's heating status, total fat content, total viable bacterial count, or whey protein levels (Phase III).
Figure 6 shows the proportion of samples above the detection limit in the advanced microbiological analyses (N=222) shown for each microbiological group stratified by milk type and milk's heating status (SM: high heat treated (N=50), SM: pasteurized (N=16), FM: heated (N=55), FM: raw (N=101)).

### Detailed description of the invention

The present invention is concerned with a raw milk preparation for use in the treatment of conditions and diseases that are caused by inappropriate immune reactions. In particular it has been found that the raw milk preparation can be used for treating or preventing asthma, and allergic diseases, in particular in infants or children.

The inventors of the present invention found that the amount of undenatured whey protein present in the raw milk preparation is inversely associated with asthma, hay fever, atopy, atopic dermatitis and food allergen sensitisation. More surprisingly, it was found that the microbial load of the raw milk preparation was not related to the clinical health outcome. The beneficial effect is obtained only if a raw milk preparation is used but the beneficial effect decreases or disappears when a thermally treated milk preparation or parts thereof are used. Furthermore, it has been found that the fat fraction of the raw milk preparation can also contribute to the beneficial effect.

Therefore the raw milk preparation of the present invention comprises at least the whey fraction and the fat or cream fraction of the milk and is preferably the whole raw milk. The raw milk preparation that is used according to the present invention comprises the heat-sensitive whey proteins of raw milk, which are present in the whey fraction of raw milk. It is of importance that those milk ingredients, which are heat-labile, such as the proteins of the raw milk that are found in whey , which can be damaged by thermal and/or other external stress, are present in native and undamaged form in the raw milk preparation used according to the present invention. Ideally, the whole fraction of heat-labile milk ingredients, such as the whey proteins, in undenatured form is present. It seems that an intact structure of the heat-labile milk ingredients, such as the whey proteins, contributes to the beneficial effect.

It was found that it is even more advantageous if the fat fraction is present in the raw milk preparation. It is contemplated that the fat globules have a role in the treatment and the structure of these globules is maintained only if no detrimental mechanical steps are applied. The globules are destroyed by mechanical stress as is applied by homogenisation.

Thus, the raw milk preparation of the present invention is in particular a raw milk preparation that has not been subjected to homogenisation, and has not been heated above 55° C for longer than 20 seconds at atmospheric pressure. A milk preparation that has been treated by heating and/or has been subjected to external stress as defined above exerted by other than thermal methods is not useful for the treatment envisaged by the present invention, as those methods lead to damage of the proteins. Thus the application of such steps shall be excluded when preparing the preparation of the present invention.

Methods like filtration or ultrafiltration, which are used to remove coarse particles, bacterial load and/or precipitated matter do not harm the heat-labile proteins or the fat globules and can be used.

The amount of heat-labile proteins can be determined with methods well-known in the art. (Whey) proteins that are denatured at temperatures beyond 55°C can be identified by analysing raw milk preparations and/or whey before and after heat treatment with enzymatic, electrophoretic, chromatographic or immunological methods.

Thus, "untreated" when used regarding raw milk means that the milk has not been subjected to thermal or mechanical treatments that lead to damage in the protein structure as outlined above, but can have been subjected to filtration and ultrafiltration, precipitation or separation of a casein fraction or any other separation method for obtaining whey proteins and/or the fat fraction provided that methods and conditions are used such that no damage to the proteins or fat globules occurs.

The beneficial effect when using the raw milk preparation of the present invention depends on the amount and frequency taken. The dosage depends on the initial composition of the milk and on the composition of the milk after processing without damaging either fat globules or heat-labile milk ingredients, such as whey proteins.

It is contemplated that the raw milk preparation of the present invention comprises 3 - 4 % proteins, of which about 20% are whey proteins. It has been found that a daily dosage of about 125 ml to about 250 ml of native raw milk or a corresponding dosage of a raw milk preparation as defined in the present application, or an amount of a freeze-dried or spray-dried powder that comprises the same amount of proteins is useful for the treatment or prevention of asthma or allergic conditions.

The raw milk preparation of the present invention is used for regular consumption, i.e. oral uptake, preferably for daily consumption. It has been shown that regular consumption of a raw milk preparation as defined in the present application is associated with a beneficial health outcome and that the effect is more evident for daily consumption. The raw milk preparation, therefore, is preferably consumed at least once a day and can be consumed more times a day, for example at every meal.

It is known that the immune system is "trained" to differentiate between heterologue and homologue in the infant's first years. Therefore, it is preferred to regularly use the raw milk preparation of the present invention for treatment of an infant or child from birth within at least 1 month, or at least three months, preferably at least until completion of the 1^{st}, even better of the 3^{rd} year of age. It has been found that regular consumption of the raw milk preparation of the present invention until the age of three could decrease substantially the susceptibility for asthma and/or allergic conditions in children.

It has been found that the raw milk preparation of the present invention can also be of benefit for the unborn infant during pregnancy and that the susceptibility for asthma and/or allergic conditions can be decreased by treating the mother during pregnancy or even in preparation for pregnancy. Thus, in another embodiment the raw milk preparation is used for treatment of a female human being during pregnancy, during nursing of the infant and/or in preparation for a planned pregnancy, as it has been shown, that it is beneficial to the health of the child, if the mother has consumed the raw milk preparation of the present invention during pregnancy or even before.

It has been found that regular intake of the raw milk preparation of the present invention is beneficial to the condition of human beings, in particular of pregnant females and children. Therefore, in a further embodiment of the present invention the raw milk preparation is used as nutritional supplement for a female adult during pregnancy, in preparation for a planned pregnancy and/or during nursing of an infant or as nutritional supplement for an infant or child in the first time of its life, such as within the first months after birth or from birth until completion of the 1^{st}, or preferably until the 3^{rd} year of age.

The raw milk preparation of the present invention can be either native raw milk or a preparation comprising at least the whey and fat fraction of raw milk as defined above or a dehydrated form thereof. The preparation can be prepared by methods known in the art. In particular any method that does not damage the heat-labile milk ingredients, such as the proteins of the whey fraction, can be used.

Thus, another embodiment of the present invention is a method of producing the raw milk preparation for use according to the present invention, wherein the method comprises removal of undesired parts from raw milk obtained by milking an animal without damaging heat-labile milk ingredients, such as the proteins in the whey fraction. In a preferred embodiment the method comprises filtration of raw milk obtained from an animal, such that a substantial amount of the whey protein fraction, such as more than about 75%, remains undenatured and wherein any heating of the milk above 55°C for longer than 20 seconds is excluded.

Milk and whey are complex systems comprising a multiplicity of components in addition to whey proteins although mostly in minute amounts. Milk is an emulsion or colloid of butterfat globules within a water-based fluid. Each fat globule is surrounded by a membrane consisting of phospholipids and proteins; these emulsifiers keep the individual globules from joining together into noticeable grains of butterfat and also protect the globules from the fat-digesting activity of enzymes found in the fluid portion of the milk.

Milk contains more than 40 proteins. Milk composition changes based on the origin of the milk, the feed of the milk animal, the race of the animal, the time in the year, and further conditions. In general cow's milk contains approximately 30 to 35 g/L (3-3.5%) of cow's milk proteins (CMPs), which can be divided into 2 main classes: Caseins (about 80%) and Whey proteins (about 20%). It has been found that the proteins, and in particular the heat-labile proteins, that are present in the whey in undamaged form are essential for the use for preventing and/or treating asthma and allergic conditions. It is assumed, without being bound by theory, that it is the native form and the combination of the proteins, particularly the heat-labile proteins, in the whey that provide for the beneficial and surprising effect of the raw milk preparation, possibly in combination with the presence of undamaged fat globules of the milk. Therefore, it is of importance that only methods for providing the raw milk preparation are used that do not or hardly change the structure and combination of the whey protein fraction and the fat fraction. Moreover, it is essential that the raw milk preparation of the present invention at least comprises the whey and fat fraction of raw milk in undenatured form and preferably is native raw milk, optionally in dehydrated form.

Whey proteins can be separated from milk caseins by precipitation using proteases like chymosin or by acidification of the milk to pH 4.6, and thereby forming the coagulum (curd). The whey proteins remain soluble in the milk serum. Thus, the raw milk preparation of the present invention comprises at least the milk serum.

In the following some of the major whey proteins are described, without being limited to these examples.

β-lactoglobulin is the most abundant protein in whey, accounting for 50% of total protein in the whey fraction. It has no homologous counterpart in human milk.

α-lactalbumin is a monomeric globular calcium-binding protein representing about 25% of whey proteins. It is a regulatory component of the enzymatic system of galactosyl transferase responsible in mammary secretory cells for the synthesis of lactose.

Bovine serum albumin (BSA) accounts for around 5% of the total whey proteins. BSA is physically and immunologically very similar to human blood serum albumin. Its main role is the transport, metabolism and distribution of ligands and the protection from free radicals.

Lactoferrin is a protein of mammary origin and is a milk-specific iron-binding glycoprotein of the transferrin family. It can be found in the milk of most species at levels lower than 1%. Lactoferrin is known to be an immunomodulatory substance. Although it is present in very low concentrations in cow's milk, it has been shown to be a strong milk allergen.

The Immunoglobulin (Ig) fraction, which includes lgG and IgE, accounts for about 1% of total milk protein and 6% of whey protein. Data on the potential allergenicity of bovine immunoglobulins are very limited. However, some studies propose IgG as another milk allergen due to the observation that IgE from cow's milk allergy patients specifically binds bovine IgG.

The proteose-peptone fraction represents about 1.1 % of the total milk protein. It is a heat-stable and acid-soluble protein fraction of milk with important functional properties. This milk component is derived mainly from the proteolysis of β-casein, and the enzymatic activity of plasmin can over time increase its concentration in milk.

### Methods of milk processing

Milk is usually processed before it is available for sale to consumers. Mostly such processes involve heat-treatment and homogenisation of milk, both treatment steps that change components in the milk. One of the main goals of conventional heat-treatment of milk is to inactivate any pathogenic microorganisms while at the same time extending its shelf-life by reducing the microbial loads of spoilage bacteria as well as enzymatic activity. However, a disadvantage of the heat treatment is degradation of the product's quality attributes and nutritional properties due to the intense heat treatment and subsequent destruction of proteins and vitamins, including undesirable changes to other characteristics such as flavour and colour.

The commonly used processes of heat treatment are not suitable for the raw milk preparation of the present invention because the most valuable part of the preparation is damaged thereby. To minimise such disadvantages, other technologies based on different preservation factors as alternatives to traditional pasteurisation are available. Any alternative method can be used as long as the heat-labile milk ingredients, such as the whey proteins, and their structure are not or hardly changed or altered.

One of these alternative methods is microfiltration. Microfiltration is widely used for the removal of microorganisms and somatic cells. It is particularly adapted to the removal of bacteria from skimmed milk, as the size of the microorganisms is in the same range as fat globules. The first membrane developments for the separation of milk components occurred in the late 1960s and have spawned a new industry for whey treatment as well as new avenues for cheese-making. Since then, membrane equipment has been adopted throughout the dairy processing chain, including milk reception, cheese-making, whey protein concentration, fractionation of protein and effluent treatment. Nowadays, 40% of the food applications of membrane processing are developed in the dairy industry, with applications and equipment serving as references for the industries treating other food liquids. Skimmed milk microfiltration makes it possible to decrease the microbial load of milk while maintaining the organoleptic quality of milk due to low heat treatment (35-55°C). The decimal reduction of bacteria ranged from 2-3 log with first ceramic membranes and reaches 3-4 log with the currently used 1.4 µm membranes. This means that microfiltered milk contains between 10 and 50 cfu/ml. The observed decimal reduction in spore counts is high: retention from 99.1 to 99.99% (2-4 log) for both aerobic and anaerobic spores or even higher reductions for specific spores such as Bacillus cereus (> 4.5 log). Decimal reduction of pathogenic bacteria *(Listeria monocytogenes, Brucella abortus, Salmonella typhimurium* and *Mycobacterium tuberculosis)* is 3.5-4.0.

After the microfiltration step or instead of it the raw milk or at least the whey and fat fraction thereof can be dehydrated by a process that does not or hardly alter the structure of the heat-labile milk ingredients, such as whey proteins. Methods for dehydration are well-known in the art. Preferred methods that are gentle and not or hardly damaging are spray drying and freeze drying.

Further processing steps are outlined below and can be applied for the preparation of the present invention provided the heat-labile milk ingredients, such as whey proteins, and the fat globules remain intact. High Pressure (HP) processing of food products generally involves the treatment of products at a pressure in the range 100-1000 MPa. HP-induced changes in milk fat have not been widely studied so far. It seems, however, that milk fat globules appear to remain intact under pressure, with no substantial changes in globule size being observed. Increased solubility of salts under pressure is highlighted particularly by the increased solubility of micellar calcium phosphate. Structural modifications of proteins under pressure have been extensively studied. Under pressure, casein micelles are disrupted, probably resulting from solubilisation of micellar calcium phosphate. The globular structure of whey proteins makes them particularly susceptible to pressure-induced denaturation. Of the whey proteins, β-lactoglobulin is the least stable to pressure and tends to unfold at pressures > 100 MPa whereas alpha-lactalbumin is considerably more stable with denaturation observed at pressure > 400 MPa. As a result of the unfolding, the reactive free sulphydryl-group of β-lactoglobulin becomes exposed and can subsequently undergo irreversible sulphydryl-disulphide interchange reactions with other surrounding molecules. It is interesting to notice that immunoglobulins that are particularly sensitive to heat-denaturation are considerably more stable to HP processing, with c.a. 90% of colostral IgG surviving treatment at 500 MPa for 5 min. Most indigenous milk enzymes are quite baroresistant. There have been numerous studies on the impact of HP on bacteria reduction in milk. It appears that in order to obtain a significant reduction (>4 log units) in the level of naturally present microbes in raw milk, treatment at 60 MPa is required.

Pulsed Electric Field (PEF) is another technique that consists of applying very short pulses (micro to milliseconds) at an electric field intensity of 10-80 kV/cm, applied to a food product held between two electrodes inside a chamber, usually at room temperature. Bacteria inactivation is achieved by changing the cell membrane permeability and is called electroporation, and is either reversible (cell membrane discharge) or irreversible (cell membrane breakdown or lysis). Depending on the experimental conditions and the microbial target selected, PEF has been shown to considerably reduce bacteria content of milk with reduction of up to 5 logs or even a total disappearance of cultivable bacteria.

Irradiation is related to the propagation of energy from the electromagnetic spectrum. Radio and television waves, microwaves, and ultraviolet and gamma rays are some of the examples of radiation included in the spectrum. During irradiation of food with ionising radiation, a cascade of secondary electrons with enough kinetic energy generates ionisation of atoms and molecules and formation of free radicals. In foods with high moisture content, chemical species are formed from the radiolysis of water. Irradiation can change some of the chemical and physical properties of milk, a result of denaturation, degradation, polymerisation, and rearrangement of proteins, vitamins and carotenoids, as well as hydroperoxide production. Results showed that solubility of proteins was reduced after irradiation treatment, which could be a result of crosslinking of protein chains; however, β-lactoglobulin antigenicity was not reduced by the treatment. Micronutrients such as minerals are not altered in a significant way in irradiated food or can even be more available. Thus, irradiation can be considered as long as conditions and wave lengths are used that do not or hardly alter the structure of the whey proteins and fat globules. Any change of the structure can be determined by methods known in the art, such as methods as outlined above.

Ohmic heating consists in the passage of an alternating electrical current through a food sample (one that has electrical conductivity), wherein the electrical resistance of the food material generates heat. The heat instantly generated inside the food is proportional to the square of the current induced, the electrical conductivity, and the type of food being heated. Because the generation of heat and its distribution throughout the material are unusually fast, the food retains its flavour and particulate integrity better than when using conventional thermal treatment. Thus, this processing step can be considered as long as conditions and heating time are such that the structure of the whey proteins and fat globules is not altered. Any change of the structure can be determined by methods known in the art, such as methods as outlined above.

For the present invention only those processing steps that do not denature proteins, in particular heat-labile proteins, can be used and those steps have to be differentiated from those steps that damage proteins and, therefore, cannot be used.

Processes that cannot be used for treatment of the raw milk or the raw milk preparation include damaging mechanical and thermal methods. A frequently used method for processing milk is homogenisation. Homogenisation is a mechanical method, wherein the milk is forced through small holes at high pressure. This breaks up the fat globules in order to spread them evenly throughout the milk and prevent separation of a cream layer. This process basically results in milk of uniform composition or consistency and palatability without removing or adding any constituents. Homogenisation increases the whiteness of milk because the greater numbers of fat globules scatter the light more effectively. Homogenized milk is not within the definition of raw milk and cannot be used in the present invention.

In contrast thereto, a filtration process is a gentle mechanical step that can be used to obtain the raw milk preparation of the present invention. In a filtration process milk or milk components are forced through ceramic microfilters, for example, to remove microorganisms. As the components of the milk are smaller than the porosity of the filters, this processing does not or hardly modify the milk and the proteins respectively, and, therefore, can be used.

It has been found that in particular the commonly used thermal methods that involve heating to more than 55°C for more than 20 s or methods and condition that have a similar or harsher effect on the proteins and/or fat globules are not suitable for the raw milk preparation of the present invention because they damage/denature the labile proteins in the whey fraction. Those methods include pasteurisation, sterilisation and ultra-heat treatment (UHT).

Pasteurisation is the most popular method of heat treatment. It is a relatively mild form of treatment, which kills harmful bacteria without significantly affecting the nutritional value or taste of the milk but alters the structure of heat-labile proteins, and, therefore, is excluded for processing of the raw milk preparation of the present invention.

The basic process for pasteurisation of whole milk involves heating the milk to a temperature of no less than 71.7°C for a minimum of 15 seconds (max 25 seconds). This process is known as High Temperature Short Time (HTST) or flash pasteurization.

Following heating, the milk is cooled rapidly to below 6°C using chilled water on the opposite side of the plate. This process also extends the keeping quality of the milk.

Sterilisation is a more severe form of heat treatment, which destroys nearly all the microorganisms present, including the vegetative cells and endospores. In a first step, the milk is pre-heated to around 50°C, then homogenised, after which it is poured into glass bottles which are closed with an airtight seal.

A gentle method of sterilisation can be achieved by direct sterilisation via steam injection, which is mainly used for sensitive products which have to be treated with short heating-holding times and high temperatures, or by indirect sterilisation, wherein the heat is transferred via heat exchangers. The indirect sterilisation is a gentle method, as the product to be sterilized does not come into direct contact with the heating medium, which can be steam, hot water or electric heat. Nevertheless, it can alter the structure of heat-labile milk ingredients, such as the whey proteins, and, therefore, is not suitable as processing step for preparing a raw milk preparation of the present invention.

Commonly, filled bottles are carried on a conveyor belt through a steam chamber where they are heated to a temperature of between 110-130°C for approximately 10-30 minutes. Then they are cooled using a cold water tank, sprays or, in some cases, atmospheric air and then crated.

The sterilisation process results in a change of taste and colour and also slightly reduces the nutritional value of the milk, particularly the B group vitamins and vitamin C, depending on the specific sterilization technology applied.

UHT or ultra-heat treated milk is a form of milk that has been heated to a temperature of at least 135°C for a very short time period in order to kill any harmful microorganisms including endospores, which may be present in the milk. The milk is then packaged into sterile containers. UHT milks have a longer shelf life as a result of the higher temperatures to which they are heated and the packaging used to store them.

All the above described heating methods that cause damage to the proteins cannot be used for the present invention.

In contrast to the above mentioned commonly used methods of heat treatment and homogenisation, an alternative approach is used for the raw milk preparation of the present invention. In other words, methods that do not harm the proteins and fat globules are used.

In another approach, raw milk is used that has been obtained by avoiding microbial contamination. Raw milk of high quality and low microbial load can be obtained, as is well-known in the art, by conforming to the safety guidelines for primary and secondary food production and consumption without conducting thermal treatment of the milk by otherwise ensuring that the milk does not contain a microorganism load which exceeds safety levels for food consumption.

A preferred form of raw milk that is used for the present invention is so-called certified raw milk, which is raw milk that has been only by filtration. Certified raw milk is obtained from cows certified as healthy. It is unpasteurized milk with a bacteria count below a specified standard. Certified raw milk can be obtained from suppliers known to the person skilled in the art. In Germany for example, certified raw milk (Vorzugsmilch) can be obtained from approximately 80 dairy farms listed by the Bundesverband der Vorzugsmilcherzeuger und Direktvermarkter von Milch & Milchprodukten. Similar organisations can be found world wide. A listing can be found on http://www.realmilk.com/where-other.html.

The present invention is further outlined and explained with reference to the examples that follow without being restricted to the embodiments outlined therein. In particular, the beneficial effects that can be obtained by using the preparation of the present invention regularly are demonstrated.

### Example 1

The GABRIEL Advanced studies were conducted in 5 rural areas of southern Germany, Switzerland, Austria, and Poland. Because of differences in study design, the Polish data will be reported separately. In Phase I a short recruitment questionnaire was distributed through elementary schools to parents of all 6-12 year old school children in the selected study areas. Three strata were defined: i) farm children, i.e. children living on a farm run by the family, ii) exposed non-farm children, i.e. children not living on a farm but regularly exposed to stables, barns or cow's milk produced on a farm, and iii) non-exposed non-farm children. For phase II analyses, a stratified random sample of 9668 was taken from 34491 eligible participants. Children whose parents had given written informed consent for blood sampling, genetic analyses, and dust sampling were eligible (Figure I). A comprehensive questionnaire (N= 8334) provided information about the participants' farm-related exposures, and 7606 also gave blood samples for IgE measurements.

For more extensive environmental sampling the study population was restricted to one center (Bavaria). Three exclusive disease strata were defined within each exposure stratum i) asthma, ii) atopy but no asthma and iii) no asthma and no atopy. Out of the 1903 eligible Bavarian children, 895 were selected applying disproportionate stratified random sampling to create equally sized samples within each of the nine strata. Milk samples of 800 subjects were analyzed. The ethics committees of the respective universities and the data protection authorities approved the study.

### Atopy

Serum immunoglobulin E levels against inhalant and food allergens were measured by fluorescence immunoassay. Atopy was defined as positive test results for specific IgE antibodies against *D. pteronyssinus,* cat, birch (cut-off 0.7 kU/L), or against a grass mix (cut-off 0.35 kU/L). Food allergy was defined as a positive fx5 test (fish, cow's milk, hen's egg, peanut, soybean, and wheat flour).

### Clinical outcomes

Health outcomes were assessed according to ISAAC standards (Beasley R, The International Study of Asthma and Allergies in Childhood (ISAAC) Steering Committee. Worldwide variation in prevalence of symptoms of asthma, allergic rhinoconjunctivitis, and atopic eczema: ISAAC The Lancet 1998; 351:1225-32). Childhood asthma was defined as either wheeze in the past 12 months, asthma inhaler use ever, or a doctor's diagnosis of asthma at least once or wheezy bronchitis more than once. Current asthma was defined as childhood asthma and wheeze in the past 12 months. Hay fever required occurrence of nasal symptoms with itchy or watery eyes in the past 12 months or a doctor's diagnosis of hay fever ever. Atopic dermatitis was defined as a doctor's diagnosis ever.

### Milk exposure assessed by questionnaire

The phase II comprehensive questionnaire gave information about the child's farm related exposures. Cow milk consumption was determined by asking whether the child consumed milk purchased at a shop (shop milk) or directly from a farm (farm milk) and whether farm milk was boiled or skimmed. Heating status of shop milk was not assessed. The parents had to indicate the life period of milk exposure from pregnancy to school age and the corresponding amounts of milk consumption. Children were grouped into the following categories i) exclusive shop milk exposure, ii) mixed milk exposure (exposure to both shop and farm milk), and iii) exclusive farm milk exposure. The information on milk boiling was used to subdivide the farm milk exposure into 'only boiled farm milk drinkers' and 'any unboiled farm milk drinkers'. The latter included children consuming exclusively unboiled farm milk as well as those consuming both unboiled and boiled farm milk. The 'any unboiled farm milk' group was further subdivided by frequency of consumption (daily unboiled farm milk vs. less than daily unboiled farm milk) and timing of first unboiled milk exposure (first exposure to unboiled farm milk in the first year of life or during pregnancy vs. after one year of age).

### Milk sample collection and analyses

In phase III, trained field workers collected cow's milk that was consumed at the participants' homes on the day of the field visit. Parents were instructed to prepare the milk as they usually did and filled out standardized milk documentation sheets. All samples were analyzed by lab staff blinded to the milk type and the health and exposure status.

Heating status of milk samples was defined by residual activity of the milk indigenous enzymes alkaline phosphatase (ALP) and lactoperoxidase (LPO) according to EC council directive 92/46/EC. Low levels of ALP (<80 mU/I) correspond to milk having been heated above 72°C for at least 15 seconds (minimum for pasteurized milk) and low levels of LPO (<20000 mU/I) correspond to milk having been heated over 85°C for at least 5 seconds (minimum for high heat treated milk). The measurements and the milk type allowed to categorize the samples as i) high heat treated shop milk (at least 85°C), ii) pasteurized shop milk (not heated over 85°C), iii) heated farm milk (at least 72°C), and iv) raw farm milk (not heated above 72°C). As 85% of the heat treated farm milk samples were heated above 85°C, all heated farm milk samples were combined for analysis. The total fat content and whey protein levels were determined for all available phase III samples.

### Microbiological Analyses

The total viable bacterial count (TVC) was assessed in all 800 milk samples and 222 samples were selected for advanced microbiological analyses using stratified random sampling (strata based on milk type, heating status, and fat content). The following microbiological groups were determined by selective plate count methods: Pseudomonades, *Enterobacteriaceae,* Micrococci plus Staphylococci, Lactobacilli, Yeast plus Mold, Bacilli plus endospores, psychrotrophic bacteria, and human pathogens.

### Statistical Analyses

All statistical analyses were performed with STATA/SE 10.1 for Windows^{TM} (College Station, TX, USA). The stratification of the study sample was taken into account by using fixed weights (weighted up to the 34491 participants eligible for phase II) and linearized Taylor series method for variance estimation. First, associations between milk exposure and health outcomes were determined in participants of phase II using weighted multivariate logistic regression models, adjusting for age, sex, farming status (farmers versus non-farmers), number of siblings, familial history of asthma or hay fever, study center, and breast feeding. In sensitivity analyses, all final models were adjusted for food allergens (fx5), asthma models were adjusted for atopy, and atopy and hay fever models were adjusted for asthma. An additional adjustment for contact to farm animals or contact to stables and barns was performed to avoid confounding by concomitant farm exposures.

The phase III data were used to explore associations between objectively assessed heating status of milk or measured milk components and asthma and atopy. These regression models were adjusted for the same set of confounders as the phase II data. Milk type and heating status were categorized into four groups with high heated shop milk as the reference category. To take into account the distribution of milk constituents with high proportions of non-detects (total viable bacterial count, lactoferrin, total IgG and bovine serum albumin), samples within the detection range were split at the median representing low and high levels while non-detects were used as reference group. Milk constituents that were measurable in all samples (α-lactalbumin, (β-lactoglobulin, TGF-β2, and fat content) were divided into tertiles with the lowest tertile as a reference group to test for linearity of the association with health outcomes. α-lactalbumin and (β-lactoglobulin were subsequently entered as continuous variables into the regression models. A factor analysis with continuous variables and varimax rotation (extraction of eigenvalues of 1.5 or higher) was used to evaluate whether the different milk constituents could be separated into different factors. Results from weighted logistic regression models were expressed as adjusted odds ratios (aOR) with corresponding 95% confidence intervals (95%-CI).

### Results

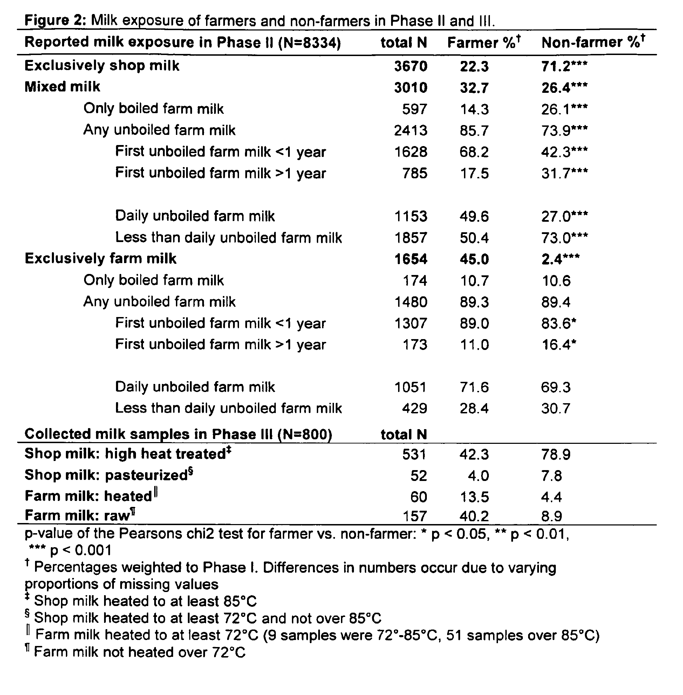

The distribution of milk consumption stratified by farm and non-farm children is shown in Figure 2. Among non- farm children, 71.2% reported exclusive shop milk consumption while 45.0% of the farm children indicated exclusive farm milk consumption. Consumption of both farm and shop milk (mixed milk exposure) were more or less comparable between farm and non-farm children respectively. The majority of farm milk consumers drank unboiled farm milk and many were exposed to unboiled farm milk already during pregnancy and or during the first year of life. Phase II questionnaire reports of milk consumption showed high agreement with the analytically determined heating status of milk samples in phase III which were collected at the participants' homes.

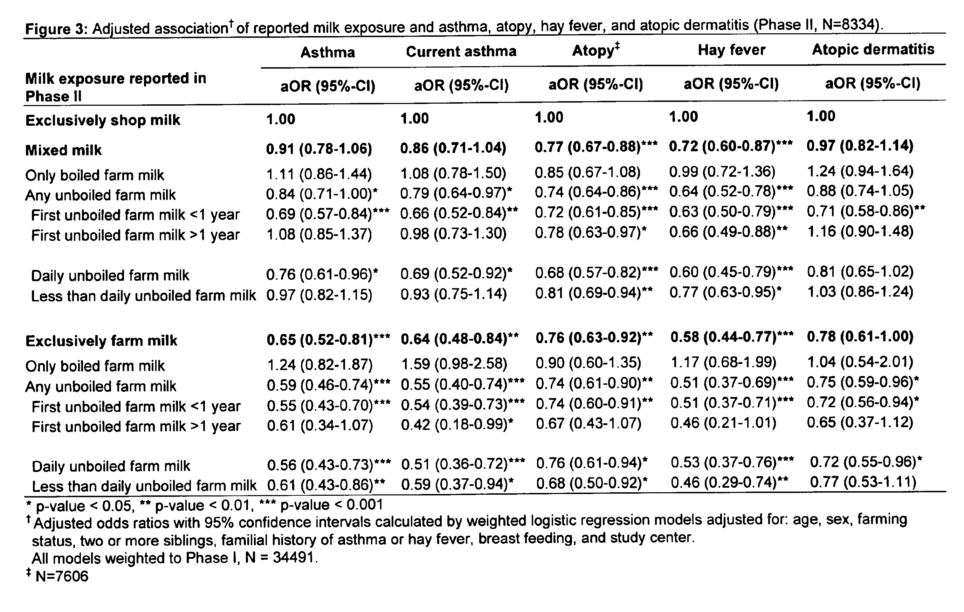

Children exclusively drinking farm milk as reported in the phase II questionnaire had significantly lower odds ratios for asthma, current asthma, atopy, and hay fever as compared to children exclusively drinking shop milk (Figure 3). The association with atopic dermatitis was of borderline significance. Mixed milk consumption (consumption of both shop and farm milk) was protective for hay fever and atopy. Consumption of any unboiled farm milk was consistently inversely associated with asthma, hay fever, and atopy in both exclusive and mixed farm milk drinkers. Early exposure and daily consumption of farm milk showed a stronger inverse association with health outcomes in mixed milk drinkers. As most exclusive farm milk drinkers were exposed to farm milk early in life with daily consumption, the power to detect the influence of frequency and age of first farm milk exposure was limited. Consumption of only boiled farm milk was not associated with any health outcome. Consumption of farm milk was also inversely related to food allergen sensitization (fx5). Compared to exclusive shop milk drinking, the association between a positive fx5 test and mixed milk consumption and exclusive farm milk drinking was (aOR (95%-Cl)) 0.85 (0.73-0.99) and 0.84 (0.69-1.03) respectively. The associations of milk consumption and asthma were robust to adjustment for atopy and food allergen sensitization.

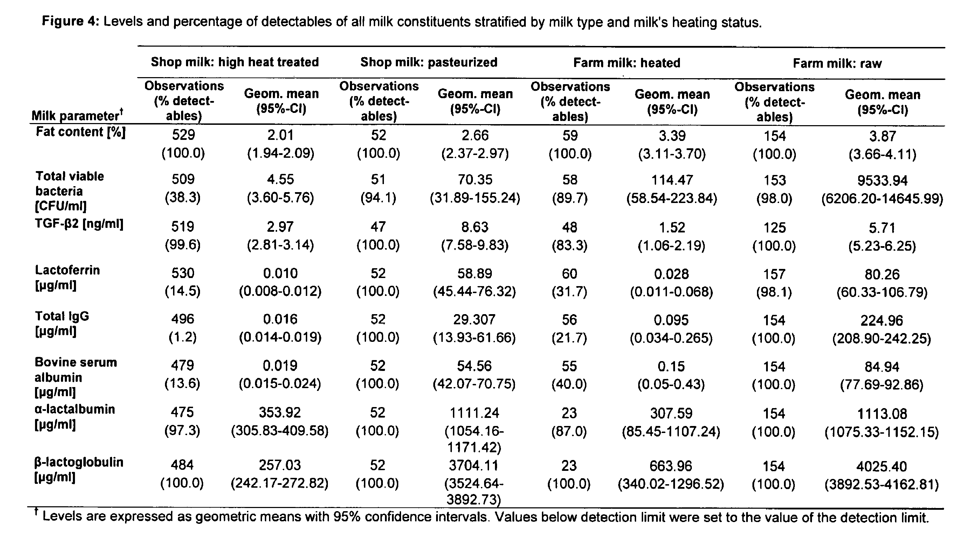

In Figure 4 total fat content, total viable bacterial count, and whey protein levels are depicted, stratified by milk type and milk's heating status. High heated shop milk showed much lower levels of all parameters as compared to raw farm milk. Heated farm milk samples had a similar fat content as raw samples but significantly lower total viable bacterial counts and lower whey protein levels (non-significant for α-lactalbumin). Pasteurized shop milk showed higher whey protein levels than high heated shop or heated farm milk.

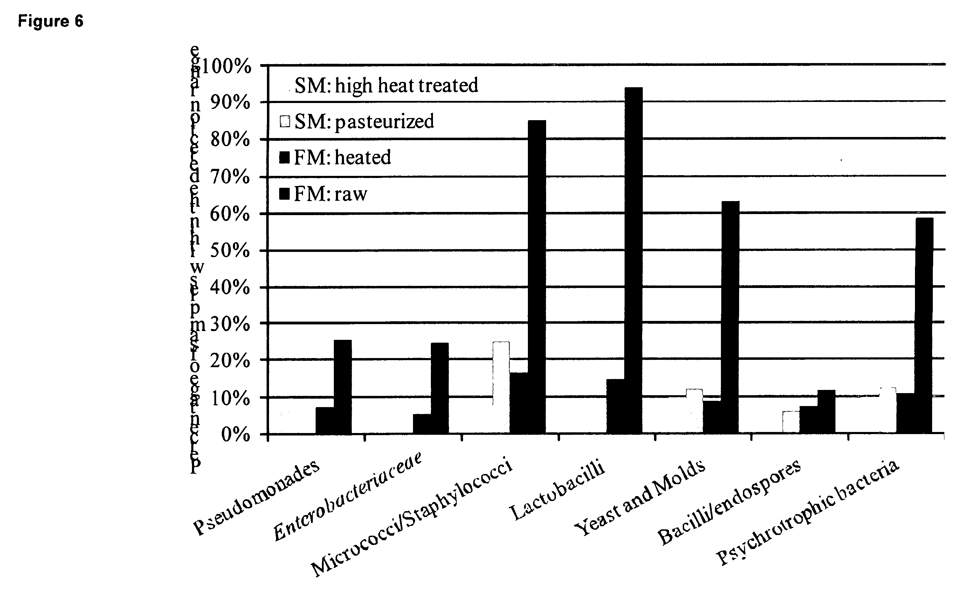

Figure 6 gives the results of the advanced microbiological analyses. In few shop milk and heated farm milk samples microorganisms could be detected (below 15% for all groups except micrococci and staphylococci (25%)). In many raw farm milk samples, micrococci and staphylococci (85.2%), lactobacilli (94.1 %), bacilli and bacterial endospores (63.4%), and psychrotrophic bacteria (58.4%) could be detected. Pathogenic *Listeria innocua* and *Listeria ivanovii* strains were found in only three unboiled farm milk samples.

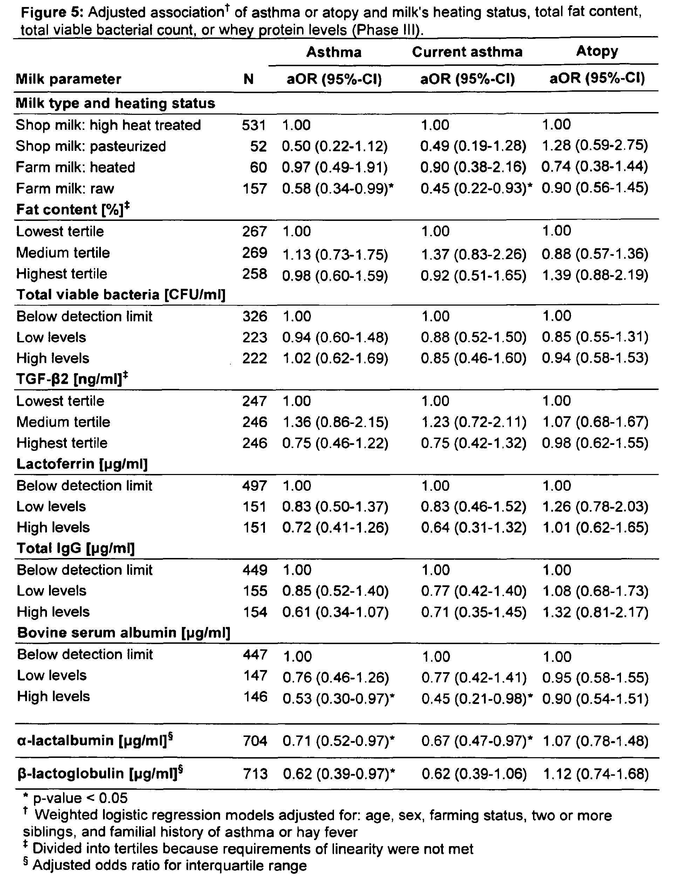

Analyses of the phase III samples (Figure 5) showed consumption of objectively assessed raw farm milk to be inversely associated with asthma (p = 0.04) and current asthma (p = 0.03) but not with atopy when compared to high heat treated shop milk. A similar risk reduction albeit not significant was observed for consumption of pasteurized shop milk and asthma. Heated farm milk was not associated with asthma outcomes. Total fat content and total viable bacterial counts had no clear association with any of the analyzed health outcomes. No association was further found between these health outcomes and total protein content, somatic cell count, lactose levels, or microbiological subgroups. Yet, increased levels of the whey proteins tended to be inversely associated with asthma but not with atopy. Statistically significant inverse associations with asthma and current asthma were found for α-lactalbumin (asthma: p = 0.03, current asthma: p = 0.03), (β-lactoglobulin (asthma: p = 0.03), and high levels of bovine serum albumin (p = 0.04, p = 0.04). Lactoferrin and total IgG showed a non significant inverse association with asthma indicative of a dose-response relation. TGF-β2 was not significantly associated with asthma or atopy although the highest tertile compared to the lowest tertile tended to be associated with a reduced asthma risk. In two exposure models including total viable bacterial counts or total fat content and individual whey proteins the results were essentially unchanged. Applying factor analysis, the different whey proteins could not be separated from each other or from milk's heating status since all were loading on the same factor.

## Claims

1. Raw milk preparation for use for treating or preventing childhood asthma and allergic diseases.

2. Raw milk preparation of claim 1 for use of claim 1, wherein the raw milk preparation is native raw milk (whole milk), or a preparation comprising at least the whey and fat fraction (whey-fat-preparation) of native raw milk, or native raw milk or a whey-fat-preparation in dehydrated form, in particular in spray dried or freeze dried form.

3. Raw milk preparation of one or more of the preceding claims for use according to claim 1, wherein the raw milk preparation is a bovine raw milk preparation.

4. Raw milk preparation of one or more of the preceding claims for use according to claim 1, wherein the childhood asthma is current and/or chronic asthma.

5. Raw milk preparation of one or more of the preceding claims for use according to claim 1, wherein the allergic disease is selected from the group consisting of hay fever, atopy and atopic dermatitis (neurodermitis).

6. Raw milk preparation of one or more of the preceding claims for use according to claim 1, wherein the raw milk preparation is part of the daily food consumption.

7. Raw milk preparation of one or more of the preceding claims for use according to claim 1 for treatment of an infant or child from birth for at least 1, or at least 3, or at least 6 months or up to completion of the 1^{st} or the 3^{rd} year of age.

8. Raw milk preparation of one or more of the preceding claims for use according to claim 1 for treatment of a female human being during pregnancy, during nursing of the infant and/or as a preparation for a planned pregnancy.

9. Raw milk preparation of one or more of the preceding claims for use according to claim 1, wherein the daily dosage of the raw milk preparation is from about 125 ml to about 250 ml of the raw milk preparation, or an amount of a freeze-dried or spray-dried powder that comprises the same amount of proteins.

10. Method of producing the raw milk preparation for use according to one or more of the preceding claims, wherein the method comprises filtration of raw milk obtained by milking of a mammal such that a substantial amount of the whey protein fraction remains undenatured, wherein heating to above 55°C for longer than 20 seconds is excluded.

11. Method of claim 11, wherein in addition to or instead of the filtration step the raw milk or raw milk preparation is dehydrated.

12. Method of claim 12, wherein dehydration is spray-drying or freeze-drying.

13. Use of the a raw milk preparation as nutritional supplement for a female human being during pregnancy, as a preparation for a planned pregnancy and/or during nursing of the infant or for an infant or child from birth to at least until completion of the 1^{st} or 3^{rd} year of age.
